# EUROPEAN PATENT SPECIFICATION

(11) **EP 3 148 443 B1**
(45) Date of publication and mention of the grant of the patent: **24.03.2021**
(21) Application number: 15755768.7
(22) Date of filing: 25.02.2015
(51) Int. Cl.: A61B 8/00, A61N 5/00, A61N 7/00, A61B 17/225, A61B 90/00

(54) **LIMITED USE ULTRASONIC COUPLING DEVICE**
BEGRENZT VERWENDBARE ULTRASCHALLKOPPLUNGSVORRICHTUNG
DISPOSITIF DE COUPLAGE À ULTRASONS À USAGE LIMITÉ

(30) Priority: 25.02.2014 US 201461944525 P
(43) Date of publication of application: 05.04.2017
(73) Proprietor: ZetrOZ Systems LLC, Trumbull, CT 06611 (US)
(72) Inventor: LEWIS, George K., Jr., Trumbull, Connecticut 06611 (US); FLESHMAN, Shane, Milford, Connecticut 06461 (US); LANGER, Matthew D., Milford, Connecticut 06460 (US)
(74) Representative: Kailuweit & Uhlemann Patentanwälte Partnerschaft mbB
(86) International application number: PCT/US2015/017596
(87) International publication number: WO 2015/130841

(56) References cited:
- WO-A2-2007/035529
- US-A1- 2008 208 084
- US-A1- 2012 277 640
- US-A1- 2013 144 193
- US-A1- 2013 144 193
- US-A1- 2014 050 321
- US-B2- 7 905 844

## Description

### FIELD OF THE INVENTION

This disclosure relates to, *inter alia*, an ultrasonic coupling device. More particularly, the present invention relates to a limited use ultrasonic coupling device as disclosed or contemplated herein. The present invention further relates to methods of using the limited use ultrasonic coupling device of the present invention.

### BACKGROUND OF THE INVENTION

Ultrasound has been used for over 60 years and is one of the most widely and frequently used electrophysical agents. Acoustic waves mechanically stimulate tissues, triggering biological effects including the down regulation of inflammatory cytokines, increased transport kinetics, protein synthesis, and extracellular matrix deposition. It has been used to treat pain, musculoskeletal injuries, and to promote soft-tissue and wound healing.

Until now, ultrasound has been primarily confined to the clinician's office, where a trained professional applies ultrasound and monitors the diagnostic, imaging, or therapeutic regimen. Therefore, devices can exist with limited safety precautions and very little to no functionality built-in for an average patient. U.S. Patent Application Publication No. 2012/0283605 discloses a portable ultrasound system that is designed to be wearable for long duration treatments. U.S. Patent Application Publication No. 2012/0277640 and U.S. Patent Application Publication No. 2013/0144193 are directed towards wearable coupling methods utilizing ultrasound coupling media. To date, other such coupling methods disclosed do not include any safety precautions against customer misuse. Furthermore, many coupling methods are not designed for adaptability to different body types and locations on the body

U.S. Patent Application Publication US 2013/0144193 A1 and U.S. Patent US 7,905,844 B2 are believed to represent the current state of the art.

The present invention incorporates mechanical, electromechanical, chemical, optical, and/or electrical means to limit the use of an ultrasound system. With self-applied, unmonitored, long duration, ultrasound coupling devices, the ultrasound coupling media will lose moisture, shrivel, or become less efficient at transmitting ultrasound as time progresses. Therefore, a means of preventing further use of inefficient coupling devices is required. This serves a two-fold purpose: coupling media that becomes less transmissive to ultrasound will (i) prevent effective ultrasound treatment and (ii) increase risk of injury to the patient by increasing thermal dissipation and thus the temperature of the coupling device.

The present invention is directed to overcoming these and other deficiencies in the art.

### SUMMARY

The present disclosure relates to ultrasound coupling adapters, ultrasound coupling devices, ultrasound coupling systems, and methods of using the adapters, coupling devices, and ultrasound coupling systems in various ultrasound applications.

In one aspect, the present disclosure provides an ultrasound coupling adapter for coupling an ultrasound transducer to an ultrasound coupling medium. The ultrasound coupling adapter comprises: an interface support region for operably interfacing the ultrasound transducer to the ultrasound coupling medium; and an integrated means for (i) rendering the ultrasound coupling adapter inoperable, and/or (ii) preventing operation of the ultrasound transducer when not properly coupled with the ultrasound coupling adapter and/or ultrasound coupling medium.

In another aspect, the present disclosure provides an ultrasound coupling device that comprises: an ultrasound coupling adapter according to the present disclosure; and an ultrasound coupling medium housed in the ultrasound coupling adapter. The integrated means is for rendering the ultrasound coupling adapter inoperable, thereby rendering the ultrasound coupling device inoperable. In certain embodiments, the ultrasound coupling device further comprises an adhesive fabric for interfacing the ultrasound coupling device with a subject, where the fabric has adhesive properties that substantially diminish after first use by the subject. In certain other embodiments, the ultrasound coupling device further comprises an adhesive fabric for interfacing the ultrasound coupling device with a subject, where the fabric has adhesive properties that diminish by 50% or greater using the ASTM D903 standard.

In another aspect, the present disclosure provides an ultrasound coupling system comprising: an ultrasound coupling device according to the present disclosure; and an ultrasound transducer configured for operable attachment to the ultrasound coupling device.

In another aspect, the present disclosure provides a method of regulating application of ultrasound energy to a subject, where the method comprises the steps of: applying ultrasound energy to a subject using an ultrasound coupling system of the present disclosure; and manipulating the integrated means of the ultrasound coupling adapter so as to render the ultrasound coupling device inoperable, thereby causing the ultrasound energy to cease being applied to the subject.

In another aspect, the present disclosure provides an ultrasound coupling device comprising: an ultrasound coupling adapter according to the present disclosure; and an ultrasound coupling medium housed in the ultrasound coupling adapter, where the integrated means is for preventing operation of an ultrasound transducer when not properly coupled with the ultrasound coupling adapter and/or ultrasound coupling medium.

In another aspect, the present disclosure provides an ultrasound coupling system comprising: an ultrasound coupling device according to the present disclosure; and an ultrasound transducer configured for operable attachment to the ultrasound coupling device.

In another aspect, the present disclosure provides a method of regulating application of ultrasound energy to a subject, where the method comprises the steps of: (i) operably coupling the ultrasound transducer to the ultrasound coupling adapter and/or ultrasound coupling medium; (ii) such that said operably coupling manipulates the integrated means; (iii) whereby the manipulation of the integrated means allows activation of the ultrasound coupling system; and (iv) applying ultrasound energy to a subject using the activated ultrasound coupling system.

In another aspect, the present disclosure provides an ultrasound coupling device comprising: an ultrasound coupling adapter according to the present disclosure; and an ultrasound coupling medium housed in the ultrasound coupling adapter, where the integrated means is for both (i) rendering the ultrasound coupling adapter inoperable and (ii) preventing operation of the ultrasound transducer when not properly coupled with the ultrasound coupling adapter and/or ultrasound coupling medium.

In another aspect, the present disclosure provides an ultrasound coupling system comprising: an ultrasound coupling device according to the aspect of the present disclosure as described in the preceding paragraph; and an ultrasound transducer configured for operable attachment to the ultrasound coupling device.

In another aspect, the present disclosure provides a method of regulating application of ultrasound energy to a subject, the method comprising the steps of: applying ultrasound energy to a subject using an ultrasound coupling system according to the aspect of the present disclosure as described in the preceding paragraph; and manipulating the integrated means of the interface support region of the ultrasound coupling device so as to (i) render the ultrasound coupling device inoperable and (ii) prevent operation of an ultrasound transducer when not properly coupled with the ultrasound coupling adapter and/or ultrasound coupling medium.

In another aspect, the present disclosure provides an ultrasound coupling device comprising: an ultrasound coupling adapter according to the present disclosure; and an ultrasound coupling medium housed in the ultrasound coupling adapter, where the ultrasound coupling device in a first state is operably connected to an ultrasound transducer and comprises an integrated means of rendering the ultrasound coupling device inoperable, and where the ultrasound coupling device in a second state is inoperable.

In another aspect, the present disclosure provides a method of regulating application of ultrasound energy to a subject, the method comprising the steps of: applying ultrasound energy to a subject using an ultrasound coupling system comprising an ultrasound transducer coupled to the ultrasound coupling device according to the aspect of the present disclosure as described in the preceding paragraph, where ultrasound energy is applied when the ultrasound coupling device is in the first state, and where ultrasound energy is not applied when the ultrasound coupling device is in the second state.

The present invention is defined in the appended claims.

### BRIEF DESCRIPTION OF THE DRAWINGS

For the purpose of illustrating aspects of the present invention, there are depicted in the drawings certain embodiments of the invention. However, the invention is not limited to the precise arrangements and instrumentalities of the embodiments depicted in the drawings. Further, as provided, like reference numerals contained in the drawings are meant to identify similar or identical elements.

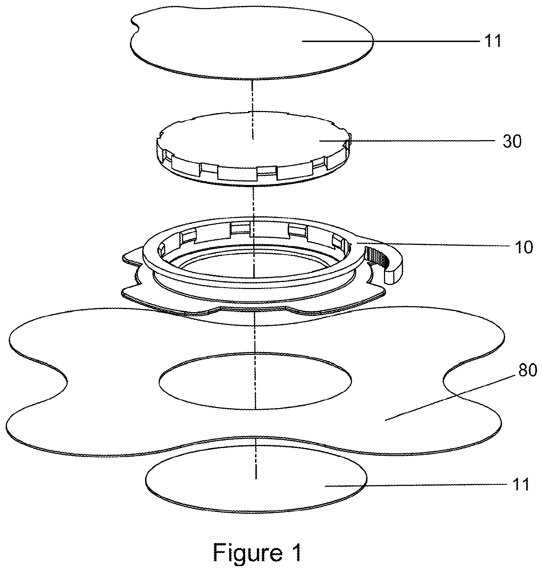

Figure 1 is an exploded view illustration showing the components comprising one possible embodiment of a limited use ultrasonic coupling device of the present disclosure. This particular embodiment resembles a bandage with a mechanical break feature to render the device unusable following treatment.

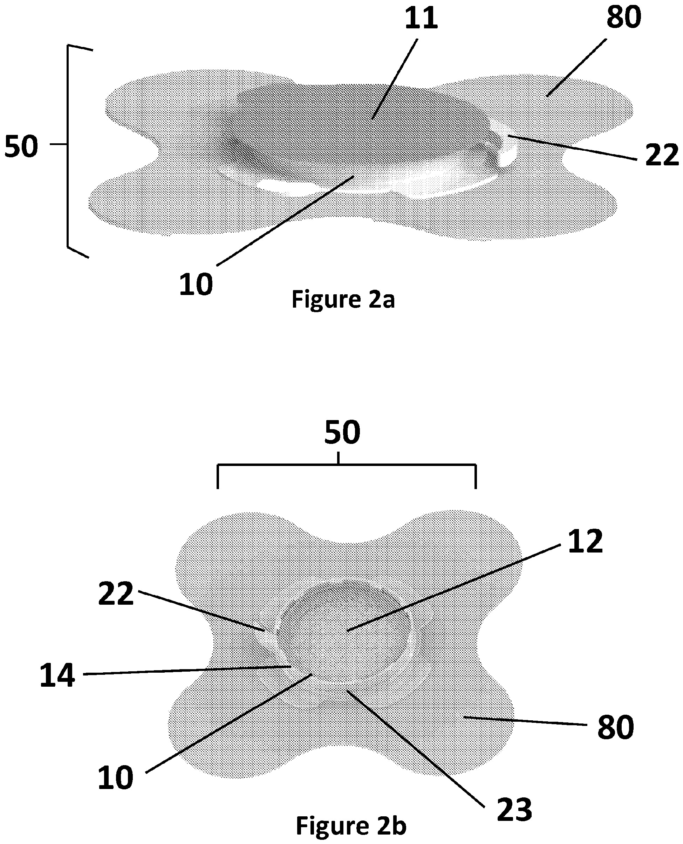

Figure 2A is a rendering showing the embodiment of Figure 1 in one configuration. Foil seals on the top and bottom of a coupling medium chamber preserve the coupling medium for proper ultrasound transmission during storage and shipping.
Figure 2B is a rendering showing the embodiment of Figure 1 with foil seals removed. In this state, the coupling device may accept an ultrasonic transducer and may be mechanically coupled to the body for therapy.

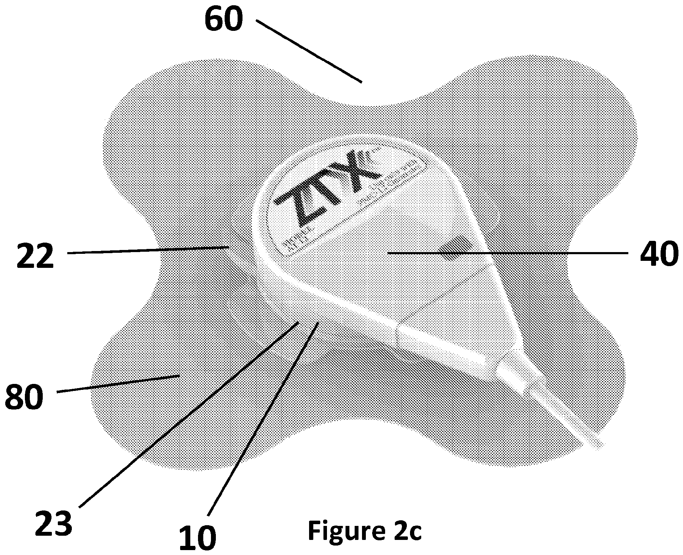

Figure 2C is a rendering showing the embodiment of Figure 1 with foil seals removed and ultrasonic transducer fastened to the coupling device. The coupling device may then be received onto a body for ultrasonic treatment.

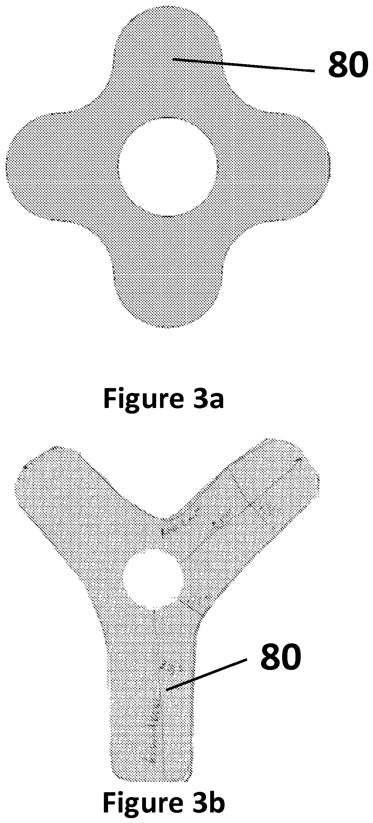

Figure 3A is an illustration depicting one embodiment of a coupling bandage. This bandage may have any shape, but is shown here with four symmetric lobes. This particular shape is beneficial for symmetric or rounded locations on a body.
Figure 3B is an illustration depicting a "Y" shaped coupling bandage used mainly in non-symmetric body locations and joints.

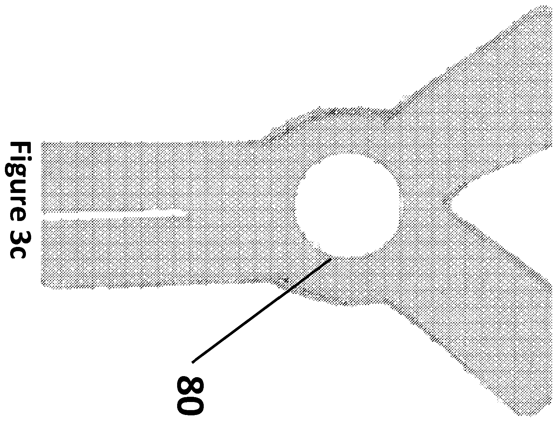

Figure 3C is an illustration depicting a modified "Y" shaped coupling bandage which may be used on joints with the bottom portion of the "Y" positioned on the articulating surfaces of the joint.

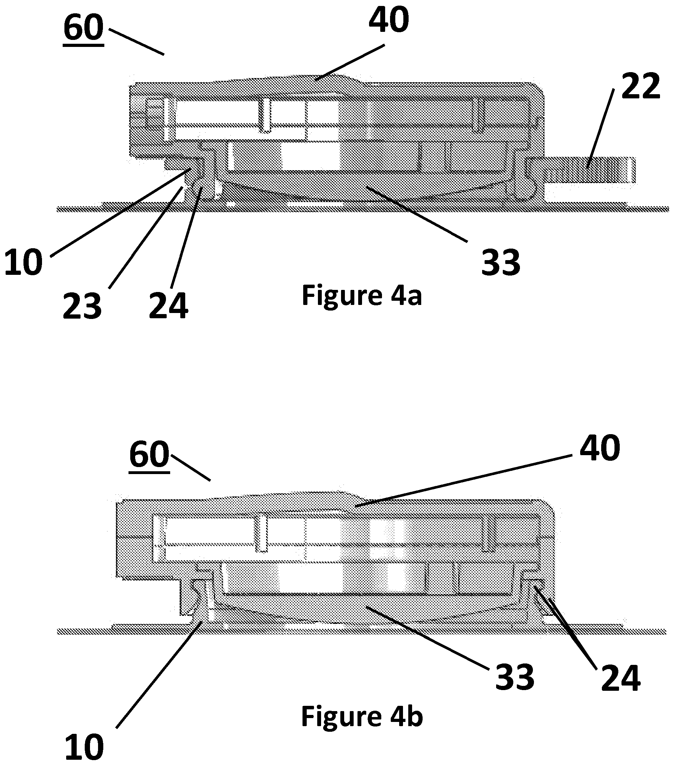

Figure 4A is an illustration showing an ultrasonic transducer mechanically fastened to an inner ring of the ultrasonic coupling device. The ultrasonic coupling device exhibits a break tab and a perforated line at the base of the coupling device for rendering the coupling device unusable following the end of a treatment.
Figure 4B is an illustration showing an ultrasonic transducer mechanically fastened to an outer ring of the ultrasonic coupling device. A simple rounded snap-fit is utilized to provide easy insertion and removal of the ultrasonic transducer. This may be modified to provide a hooked snap-fit for difficult removal of the ultrasonic transducer without damaging the coupling device.

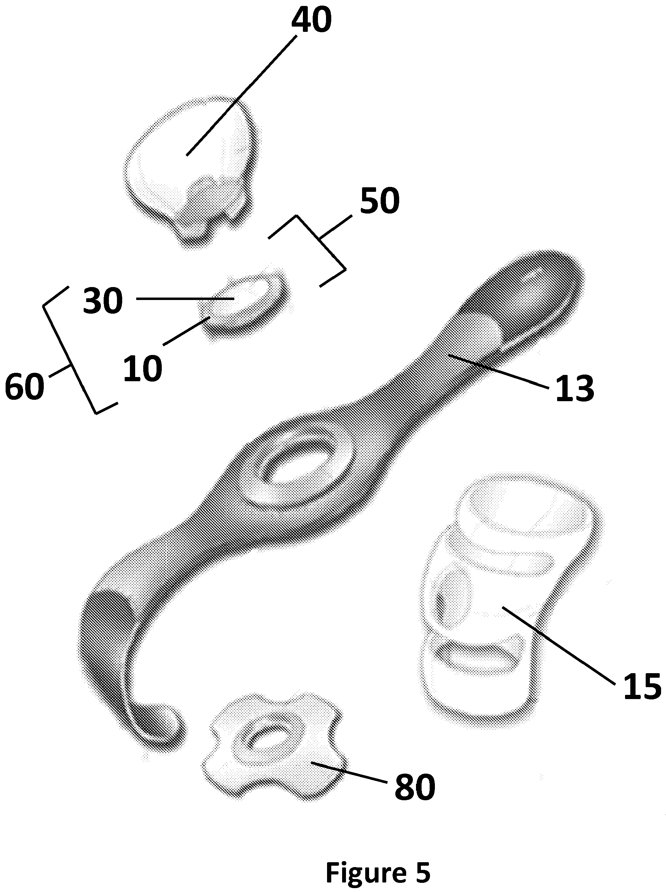

Figure 5 is an illustration of a gel puck. The gel puck may be mechanically coupled to the ultrasonic transducer and the ultrasonic transducer mechanically coupled to a wrap, brace, bandage, or other fixture. The gel puck may also be mechanically coupled directly to the wrap, brace, bandage, or other fixture. A disposable mechanism can be applied to the gel puck and or fixture.

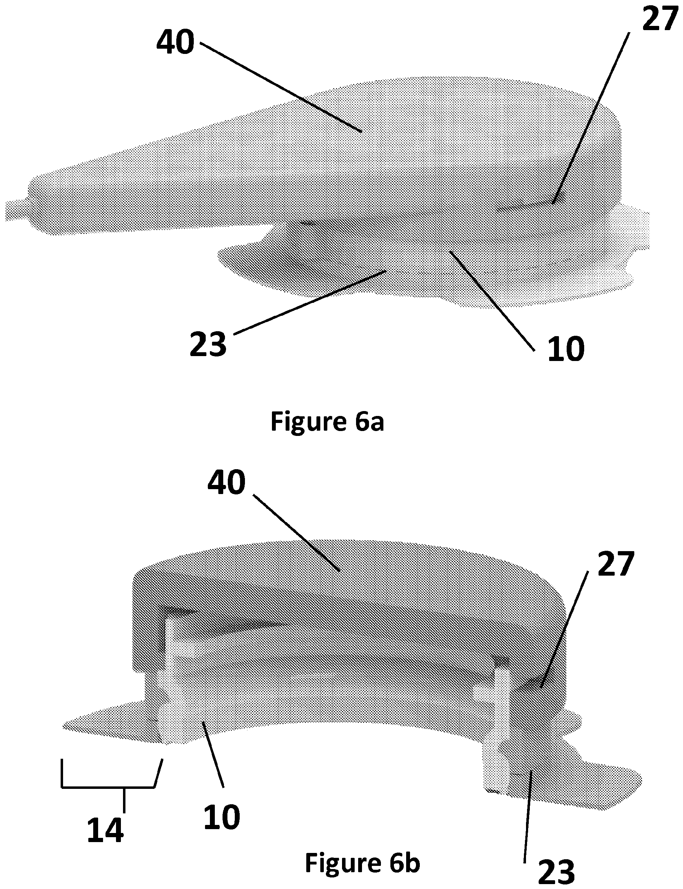

Figure 6A is a rendering of one embodiment of the present invention. An ultrasonic transducer is mechanically coupled, via snap fit, to a coupling device which has a thinned or perforated area. The mechanical fit is such that prevents removal of the ultrasonic transducer without damaging the coupling device. Following a treatment, the ultrasonic transducer may be twisted to break the thinned or perforated area, which releases the ultrasonic transducer from the coupling device and renders the coupling device unusable.
Figure 6B is a rendering of the cross-section of the embodiment described in Figure 6A. The cross-section shows the one-way mechanical snapping feature of the coupling device. The cross-section further shows the perforated area and a slotted area in the ultrasonic transducer that interacts with a boss on the coupling device to deliver a shear force on the perforated area.

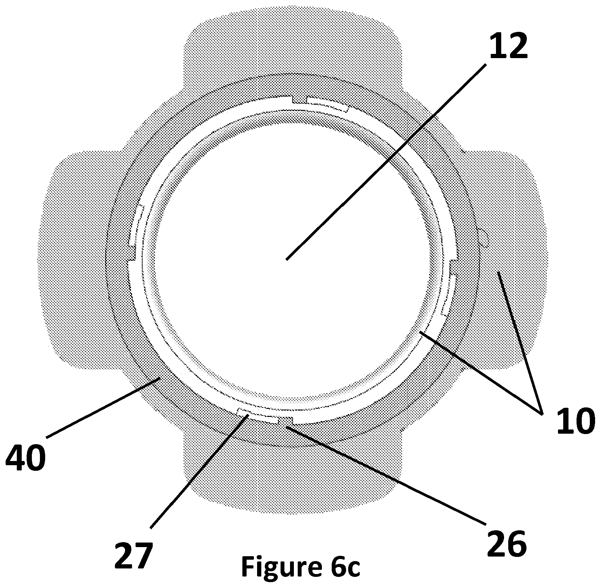

Figure 6C is a rendering of another cross-section of the ultrasonic coupling device embodiment described in Figure 6A. This cross-section shows the interaction of the ultrasonic transducer with the coupling device in the slotted regions. This interaction creates the shear force needed to break the perforated area of the coupling device.

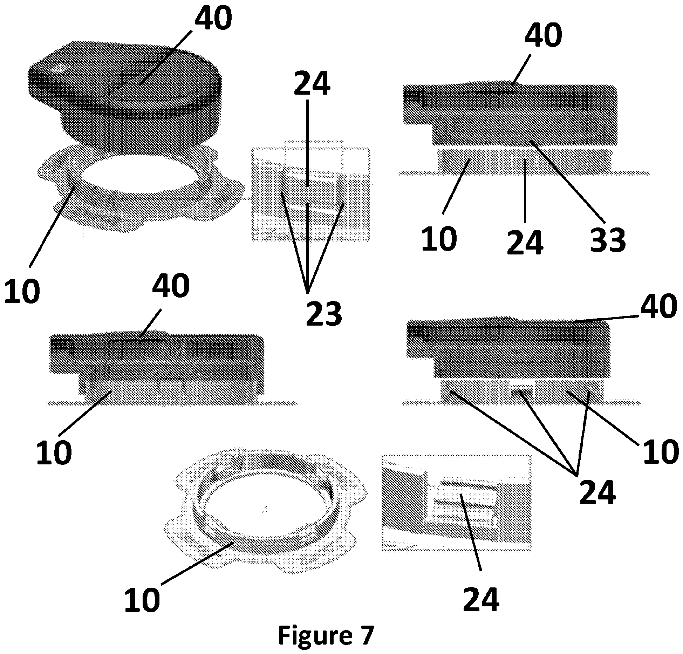

Figure 7 shows a series of renderings of one embodiment of the ultrasonic coupling device. This embodiment utilizes four areas for mechanically fastening an ultrasonic transducer to the coupling device. These four areas are surrounded by thinned or mechanically weak areas. When the ultrasonic transducer is removed from the coupling device, the upward pull force is converted into a transverse force which breaks the thinned areas surrounding the mechanical fasteners. This allows removal of the ultrasonic transducer and renders the ultrasonic coupling device unusable by preventing subsequent ultrasonic transducer connections.

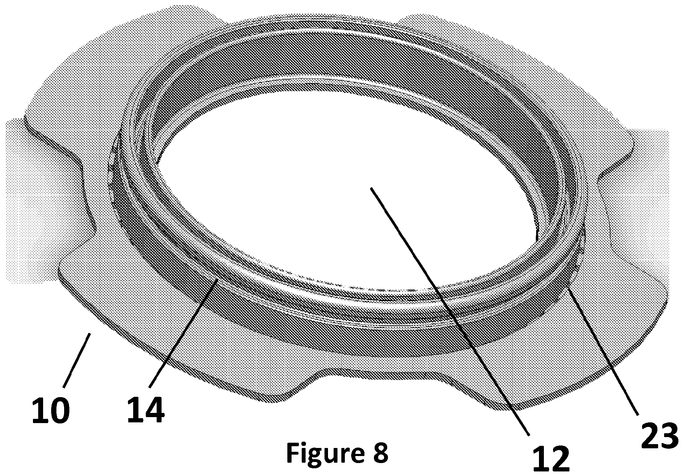

Figure 8 shows a rendering of one embodiment of the present invention. This embodiment resembles that of Figure 6; however the perforated or thinned area is at the intersection of the coupling medium chamber and the patient coupling area. When the perforation is broken, the coupling chamber can no longer be held to the patient and the coupling device is rendered unusable.

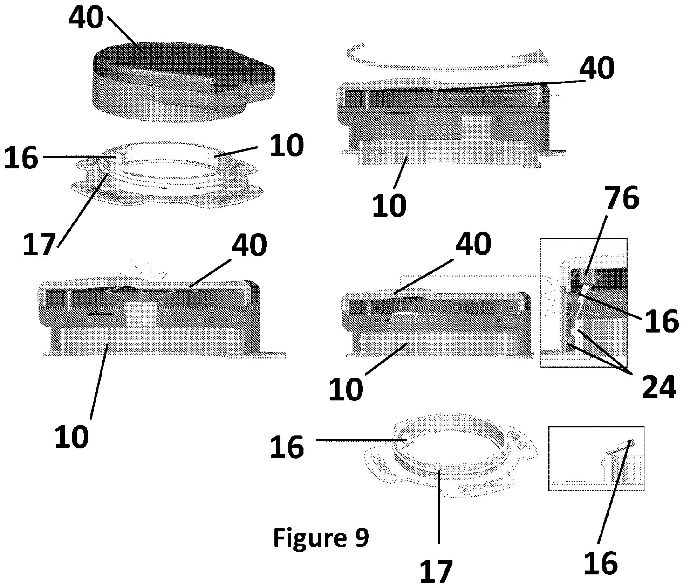

Figure 9 is a series of renderings of one embodiment of the present disclosure that utilizes an electromechanical method. An ultrasonic transducer is fastened onto the coupling device, in this case, with a screw threading. Upon turning the ultrasonic transducer completely onto the coupling device, an internal boss of the ultrasonic transducer causes an angled boss of the coupling device to engage an electromechanical switch that allows ultrasonic treatment to begin.

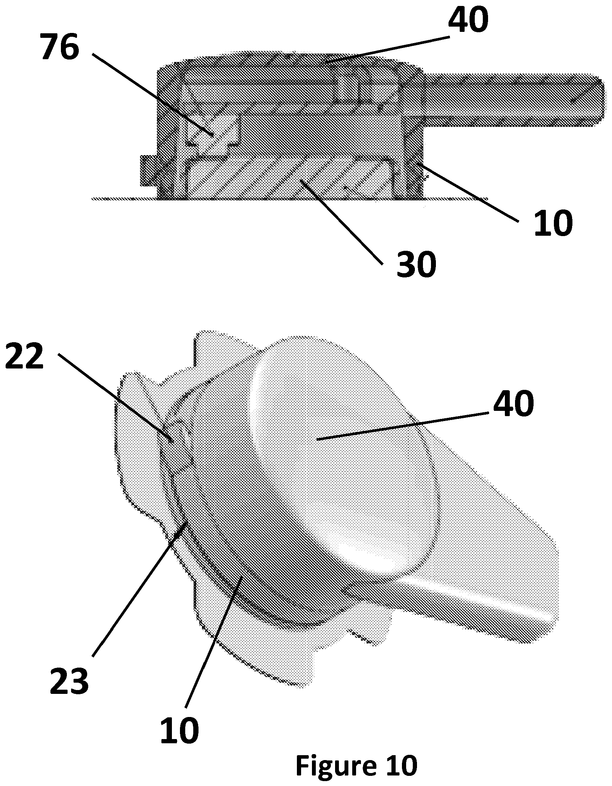

Figure 10 is a drawing of one embodiment of the present disclosure that utilizes an electromechanical means of activating and limiting the use of the ultrasonic treatment. A tactile switch is mounted on the ultrasonic transducer. The ultrasonic transducer is then mechanically fastened to the coupling device. The tactile switch is activated by a rigid or semi-rigid coupling medium that allows activation of the ultrasonic treatment. Software controls can ensure that the device is removed following treatment completion.

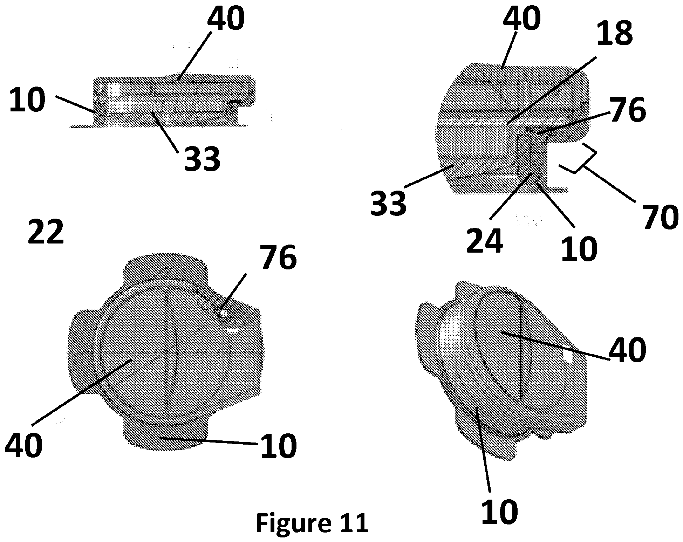

Figure 11 is a drawing of one embodiment of the present disclosure that utilizes an electromechanical means of activating and limiting the use of the ultrasonic treatment. A tactile switch is mounted on the ultrasound ultrasonic transducer. When the ultrasonic transducer is mechanically fastened to the coupling device, the tactile switch is depressed by the support wall of the ultrasound coupling medium chamber, which activates the ultrasonic treatment.

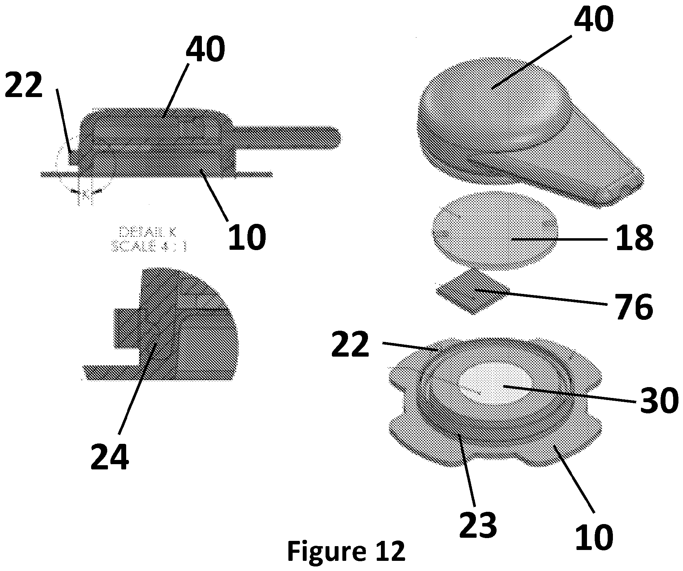

Figure 12 is a series of renderings of one embodiment of the present disclosure that utilizes an electromechanical means of activating and limiting the use of the ultrasonic treatment. A membrane switch is placed between the PCB and ultrasound transducer within the ultrasound ultrasonic transducer. The ultrasonic transducer is depressed slightly when mechanically fastened to the coupling device, which depresses the internal membrane switch and activates the ultrasonic treatment.

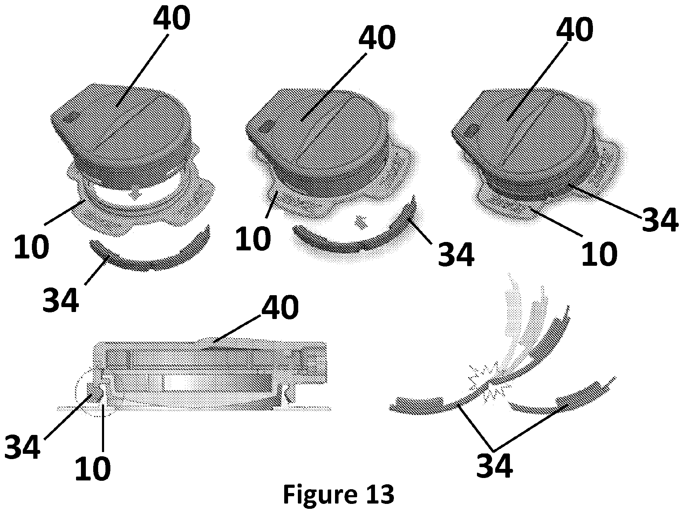

Figure 13 is a series of renderings of one embodiment of the present disclosure that utilizes an electromechanical means of activating and limiting the use of the ultrasonic treatment. Once the ultrasonic transducer is mechanically fastened to the coupling device, a secondary connector is inserted, which completes a circuit and activates the ultrasonic treatment.

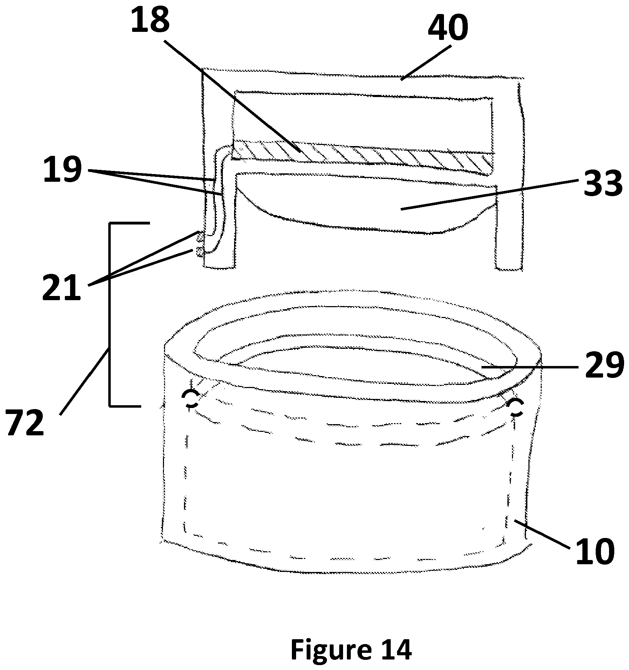

Figure 14 is an illustration depicting a coupling device with embedded electrically conductive ring. An ultrasonic transducer with two external conducting terminals contact the coupling device conductive ring when fully coupled together. This completes a circuit and activates the ultrasonic treatment.

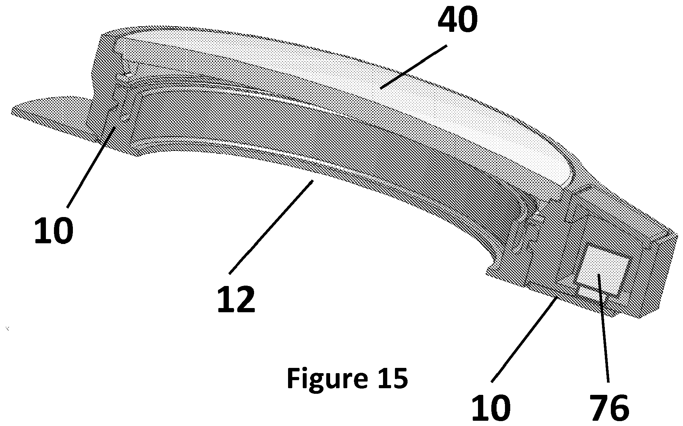

Figure 15 is an illustration depicting one embodiment of the present disclosure that utilizes an electromechanical means of activating and limiting the use of the ultrasonic treatment. A tactile switch is mounted inside the ultrasound ultrasonic transducer. When the ultrasonic transducer is mechanically fastened to the coupling device, the tactile switch is depressed by the area of the ultrasound coupling device used for coupling to a patient's body. Depressing the switch allows activation of the ultrasonic treatment.

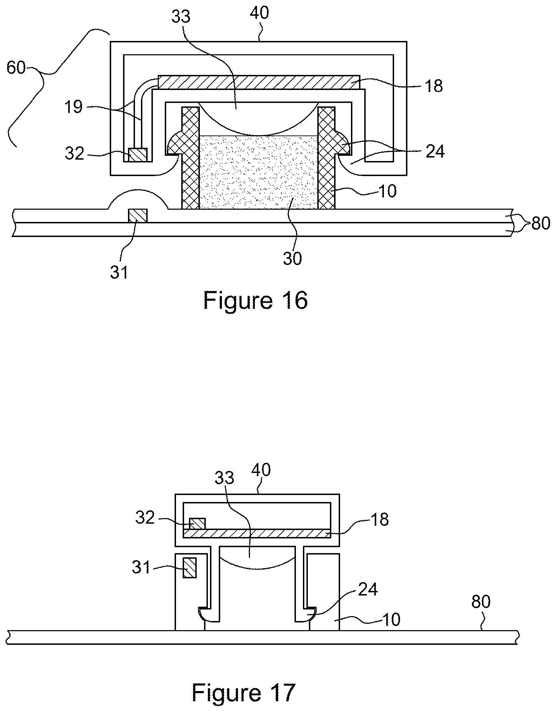

Figure 16 is an illustration that depicts a passive or active electrical component implanted within the fabric of the coupling device. The electrical component(s) comprise a means of wireless communication (e.g. RFID, NFC, BLE) from the coupling device to the ultrasonic transducer.
Figure 17 is an illustration that depicts a passive or active electrical component implanted within the ultrasonic coupling medium chamber. The electrical component(s) comprise a means of wireless communication (e.g. RFID, NFC, BLE) from the coupling device to the ultrasonic transducer.

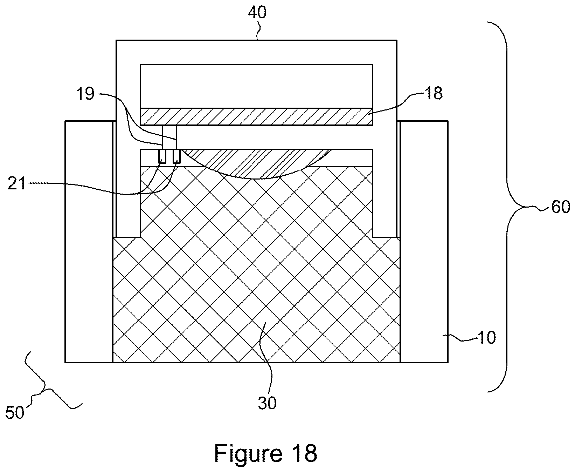

Figure 18 is an illustration of a coupling device that contains an ultrasonic coupling medium with a narrow impedance spectrum. An impedance analyzer within the ultrasonic transducer is brought into contact when the ultrasonic transducer is coupled to the coupling device. The impedance of the coupling medium may be continuously or periodically monitored to ensure proper acoustic coupling and safety.

### DETAILED DESCRIPTION OF THE INVENTION

The present invention relates to an ultrasound coupling adapter, an ultrasound coupling device comprising the ultrasound coupling adapter, and an ultrasound coupling system comprising the ultrasound coupling device and an ultrasound transducer, as further described herein. The present invention also relates to various ultrasound kits and ultrasound coupling systems configured to include the ultrasound coupling adapter and device of the present invention. Further, the present invention relates to various methods of using and making the ultrasound coupling adapter, device, and system of the present invention.

The ultrasound coupling adapters, devices, and systems of the present invention have various attributes, as described more fully herein. In particular embodiments, the ultrasound coupling adapter, device, and system are configured for limited use or one-time use. Without meaning to limit the present invention to a particular embodiment, provided below are various attributes of the present invention.

In one aspect, the present invention provides an ultrasound coupling adapter for coupling an ultrasound transducer to an ultrasound coupling medium. As used herein, the term "ultrasound coupling adapter" may also be referred to as an "ultrasound coupling compartment" or other terms that one of ordinary skill in the art would understand as referring to the "ultrasound coupling adapter" as more fully described below. The ultrasound coupling adapter comprises: an interface support region for operably interfacing the ultrasound transducer to the ultrasound coupling medium; and an integrated means for (i) rendering the ultrasound coupling adapter inoperable, and/or (ii) preventing operation of the ultrasound transducer when not properly coupled with the ultrasound coupling adapter and/or ultrasound coupling medium.

As used herein, the term "interface support region" refers to any structure that is effective for operably interfacing an ultrasound transducer to an ultrasound coupling medium. In certain embodiments, the interface support region can be configured as a single component. However, in other embodiments, the interface support region can be made of multiple components that, when combined, are effective for operably interfacing an ultrasound transducer to an ultrasound coupling medium.

Without intending to limit the scope of the invention, in certain embodiments, the interface support region has a dual purpose of providing a housing region for the ultrasound coupling medium, and providing a structural support region for contacting with and/or positioning the ultrasound transducer for operable interfacing with the ultrasound coupling medium. In such embodiments, the "housing region" functions to locate, orient, position, and/or partially or fully constrain the ultrasound coupling medium for purposes of interfacing with the ultrasound transducer. Examples of suitable interface support regions are illustrated in various drawings of the present disclosure, as further described below.

As used herein, the term "housing region" may also be referred to as an "inner chamber" or "compartment" or similar such term. In such embodiments, a term such as "inner chamber" is meant to describe an area within the inner boundaries of the interface support region, with the interface support region providing a solid structure or boundary for housing the ultrasound coupling medium.

In another aspect, the present invention provides an ultrasound coupling device that comprises: an ultrasound coupling adapter according to the present disclosure; and an ultrasound coupling medium housed in the ultrasound coupling adapter. The integrated means is for rendering the ultrasound coupling adapter inoperable, thereby rendering the ultrasound coupling device inoperable. In certain embodiments, the ultrasound coupling device further comprises an adhesive fabric for interfacing the ultrasound coupling device with a subject, where the fabric has adhesive properties that substantially diminish after first use by the subject. In certain other embodiments, the ultrasound coupling device further comprises an adhesive fabric for interfacing the ultrasound coupling device with a subject, where the fabric has adhesive properties that diminish by 50% or greater using the ASTM D903 standard.

In one embodiment of the ultrasound coupling device of the present disclsosure, the integrated means functions to permanently alter the interface support region or the ultrasound coupling medium. In certain embodiments, the integrated means is configured so that permanently altering the interface support region is achieved by bending, twisting, tearing, pulling, or pushing a feature of the integrated means.

In one embodiment, the integrated means is selected from the group consisting of a pull tab, a perforated or thinned area, a snap-fit tab, and a stress concentrator, said integrated means being configured to facilitate permanently altering a portion of the interface support region.

In one embodiment, the pull tab serves as a lever capable of rendering the ultrasound coupling device inoperable.

In one embodiment, the perforated or thinned area completely or partially extends around the perimeter of the outer support region of the ultrasound coupling adapter.

In one embodiment, the interface support region comprises a key and keyseat pair configuration with an ultrasound transducer that interface together to facilitate the permanent altering of the configuration.

In one embodiment, the interface support region comprises a threaded portion that couples with an ultrasound transducer, where the threaded portion prevents decoupling with the ultrasound transducer unless at least a portion of said interface support region is permanently altered.

In one embodiment, the perforated or thinned areas are adjacently disposed to the snap-fit tab that enable permanent deformation of the configuration upon insertion or removal of an ultrasound transducer.

In one embodiment, the integrated means comprises thermal, chemical, or ultrasound-assisted degradation. In certain embodiments, the degradation is controlled by the thickness and chemical composition of the interface support region or ultrasound coupling medium.

In another aspect, the present invention provides an ultrasound coupling system comprising: an ultrasound coupling device according to the present disclosure; and an ultrasound transducer configured for operable attachment to the ultrasound coupling device.

In another aspect, the present invention provides a method of regulating application of ultrasound energy to a subject, where the method comprises the steps of: applying ultrasound energy to a subject using an ultrasound coupling system of the present disclosure; and manipulating the integrated means of the ultrasound coupling adapter so as to render the ultrasound coupling device inoperable, thereby causing the ultrasound energy to cease being applied to the subject. In one embodiment of this method, the manipulating step is performed by the subject or triggered by the integrated means itself.

In another aspect, the present invention provides an ultrasound coupling device comprising: an ultrasound coupling adapter according to the present disclosure; and an ultrasound coupling medium housed in the ultrasound coupling adapter, where the integrated means is for preventing operation of an ultrasound transducer when not properly coupled with the ultrasound coupling adapter and/or ultrasound coupling medium.

The integrated means can comprise an electromechanical, electrical, or optical means.

In one embodiment, the electromechanical means comprises a switch selected from the group consisting of a slide, toggle, rotary, rocker, knife, pushbutton, and membrane. According to certain embodiments, the switch is located on or within the ultrasound transducer and is positioned to interact with the ultrasound coupling adapter, the ultrasound coupling medium, the subject, or the ultrasound transducer, or with an enclosure or lens in which the ultrasound transducer is affixed.

In one embodiment of the ultrasound coupling device, the electrical means functions by completing a circuit by connecting contacts located on an ultrasound transducer using a metallic or other electrically conductive material.

The electrically conductive material can be constructed to at least partially extend around the perimeter of the interface support region of the ultrasound coupling adapter.

In one embodiment of the ultrasound coupling device, the optical means functions by interrupting or completing an optical switch when the ultrasound transducer is mechanically connected to the ultrasound coupling device.

In another aspect, the present invention provides an ultrasound coupling system comprising: an ultrasound coupling device according to the present disclosure; and an ultrasound transducer configured for operable attachment to the ultrasound coupling device.

In another aspect, the present invention provides a method of regulating application of ultrasound energy to a subject, where the method comprises the steps of:
(i) operably coupling the ultrasound transducer to the ultrasound coupling adapter and/or ultrasound coupling medium, such that said operably coupling manipulates the integrated means, whereby the manipulation of the integrated means allows activation of the ultrasound coupling system; and (ii) applying ultrasound energy to a subject using the activated ultrasound coupling system. In one embodiment of this method, the manipulating step is performed by the subject or triggered by the integrated means itself.

In another aspect, the present invention provides an ultrasound coupling device comprising: an ultrasound coupling adapter according to the present disclosure; and an ultrasound coupling medium housed in the ultrasound coupling adapter, where the integrated means is for both (i) rendering the ultrasound coupling adapter inoperable and (ii) preventing operation of the ultrasound transducer when not properly coupled with the ultrasound coupling adapter and/or ultrasound coupling medium.

The integrated means of this ultrasound coupling device can comprise an electromechanical, electrical, or optical means.

In one embodiment, the electrical means comprises either passive or active components selected from the group consisting of a radio frequency identification (RFID) tag, a near field communication (NFC) tag, a Bluetooth module, a Bluetooth low energy (BLE) module, a Wireless Fidelity (Wi-Fi) module, a ZigBee module, a cellular module, or other wireless technologies, with supporting electronics selected from the group consisting of batteries, memory, microcontrollers, field programmable gate arrays (FPGA), and programmable logic devices (PLD).

In one embodiment, the electrical means includes a passive or active radio frequency identification (RFID) or Near Field Communication (NFC) tag that contain either a unique or generalized access code that is received by a reader located in an ultrasound system containing the device.

In one embodiment, the tag access code is erased following commencement or termination of an ultrasound treatment of the subejct.

In one embodiment, the tag is embedded within or adhered to the interface support region of the ultrasound coupling adapter, the ultrasonic coupling medium, or an adhesive fabric in close proximity to the tag reader when an ultrasound transducer is mechanically connected to the ultrasound coupling device.

In one embodiment, the electrical means includes an ultrasound transducer that directly or indirectly determines the electrical impedance of the ultrasound coupling medium through electrical contacts.

In one embodiment, the device functions with an ultrasound system that includes a barcode, Quick Response (QR) code, or similar reader, an infrared sensor, or an optical sensor.

In one embodiment, the optical means functions to scan a unique access code by an ultrasound system in the form of a barcode or Quick Response (QR) code located on the ultrasound coupling device, permit system operation, and restrict system operation after a predetermined period of time.

In one embodiment, the optical means comprises an optical sensor that measures the color or opacity of the ultrasound coupling medium.

In one embodiment, the ultrasound coupling medium is compounded with an oxygen or ultrasound-sensitive molecule that changes the color or opacity of the ultrasound coupling medium.

In one embodiment, the optical means comprises an infrared sensor that measures the rate of change and absolute value of temperature of the ultrasound coupling medium.

In one embodiment, the optical means comprises an optical sensor that measures the index of refraction or back scatter of the ultrasound coupling medium.

In another aspect, the present invention provides an ultrasound coupling system comprising: an ultrasound coupling device according to the aspect of the present disclosure as described above; and an ultrasound transducer configured for operable attachment to the ultrasound coupling device.

In another aspect, the present invention provides a method of regulating application of ultrasound energy to a subject, the method comprising the steps of: applying ultrasound energy to a subject using an ultrasound coupling system according to the aspect of the present disclosure as described above; and manipulating the integrated means of the outer support region of the ultrasound coupling device so as to (i) render the ultrasound coupling device inoperable and (ii) prevent operation of an ultrasound transducer when not properly coupled with the ultrasound coupling adapter and ultrasound coupling medium. In one embodiment, the manipulating step is performed by the subject or triggered by the integrated means itself.

In another aspect, the present invention provides an ultrasound coupling device comprising: an ultrasound coupling adapter according to the present disclosure; and an ultrasound coupling medium housed in the ultrasound coupling adapter, where the ultrasound coupling device in a first state is operably connected to an ultrasound transducer and comprises an integrated means of rendering the ultrasound coupling device inoperable, and where the ultrasound coupling device in a second state is inoperable.

In another aspect, the present invention provides a method of regulating application of ultrasound energy to a subject, the method comprising the steps of: applying ultrasound energy to a subject using an ultrasound coupling system comprising an ultrasound transducer coupled to the ultrasound coupling device according to the aspect of the present disclosure as described above, where ultrasound energy is applied when the ultrasound coupling device is in the first state, and where ultrasound energy is not applied when the ultrasound coupling device is in the second state.

In one embodiment of this method, first and second states are achieved by an action performed by the subject or triggered by the ultrasound coupling device.

The drawings referred to herein are for the purposes of illustrating the various aspects of the present invention and are not meant to limit the scope of the present invention. Below is a description of aspects and embodiments of the present invention as illustrated in the accompanying drawings.

Referring now to Figure 1 in particular, as well as to various aspects of Figures 1-18, there is shown an exploded view illustration of one embodiment of an ultrasound coupling device **50** comprising a top foil seal **11,** an ultrasound coupling medium **30,** an ultrasound coupling adapter **10,** an adhesive fabric **80,** and a bottom foil seal **11.** The top and bottom foil seals **11** seal to the ultrasound coupling adapter **10** to isolate the ultrasound coupling medium **30** with a water-tight barrier from the atmosphere. The foil seal **11** may be composed of, but not limited to, aluminum, tin, stainless steel, or copper. The foil seal **11** may be co-laminated with various other materials that may be composed of, but not limited to, polyethylene, polypropylene, wax, polyethylene terephthalate, polyimide, acrylic, or silicone. These co-laminates allow bonding to the ultrasound coupling adapter **10** based on the material composition of the ultrasound coupling adapter **10.** The bonding to the ultrasound coupling adapter **10** and/or to the adhesive fabric **80** may be accomplished through induction or conduction heat seal, ultrasound welding, friction welding, or laser beam welding. The adhesive fabric **80** may be composed of, but not limited to, a non-woven polymer fabric, woven fabric, elastomeric fabric, polyester fabric, polypropylene fabric, rayon, nylon, or other synthetic materials, or polyurethane foam.

The ultrasound coupling medium **30** may be comprised of water, ultrasound gel, or hydrogel. The ultrasound coupling medium **30** is designed to transmit ultrasound effectively at frequencies ranging from 20 kHz to 40 MHz and may be cured ex situ, in situ, or may not require curing. The ultrasound coupling medium **30** is designed such that it contacts the ultrasound transducer **40** (see, e.g., Figure 2C) and surface of a patient's body in the area requiring treatment, imaging, or diagnosis. The coupling of an ultrasound transducer **40** to the ultrasound coupling device **50** (see, e.g., Figures 2A-2C) can protrude the ultrasound coupling medium **30,** which allows coupling to concave portions of a patient's body.

As shown in Figures 1-4, the ultrasound coupling adapter **10** serves multiple functions, it: mechanically connects the ultrasound coupling device **50** to the ultrasound transducer **40** by way of an interface support region **14,** maintains the shape of the ultrasound coupling medium **30,** provides sealing surfaces for the foil seals **11,** and may or may not provide a means for dislodging the ultrasound transducer **40** that renders the ultrasound coupling device **50** inoperable following treatment termination. In one embodiment, the interface support region **14** comprises snap fit tabs **24** on the internal ring of the ultrasound coupling adapter **10.** The snap fit tabs **24** may be designed for two-way or one-way operation, that is, removable or permanent connection to the ultrasound transducer **40,** respectively. With a one-way connection of the ultrasound coupling adapter **10** to the ultrasound transducer **40,** the ultrasound coupling adapter **10** includes a pull tab **22.** The pull tab **22** may be any shape or size with pull direction tangential, radial, horizontal, or vertical, and provides leverage for a patient to break the ultrasound coupling adapter **10.** In one embodiment, the pull tab **22** is curved with a thinned or perforated area **23** underneath the pull tab **22.** The thinned or perforated area **23** may continue partially or completely around the circumference or perimeter of the ultrasound coupling adapter **10.** Upon applying force to the pull tab **22,** a shear force is generated in the thinned or perforated area **23** causing the material to separate and ultrasound coupling adapter **10** to dislodge the ultrasound transducer **40.** The breaking of the ultrasound coupling adapter **10** prevents future ultrasound transducers **40** from mounting to the ultrasound coupling adapter **10** and thus prevents old or dried ultrasound coupling medium **30** from being improperly used in treatment.

In another embodiment, two perforated or thinned areas **23** may be located above and below the pull tab **22.** When applying force to the pull tab **22,** a shear force is generated in the thinned or perforated areas **23** and causes both areas to split. In this embodiment, the whole structure is not destroyed, but just a portion of the ultrasound coupling adapter **10.** The portion of the ultrasound coupling adapter **10** destroyed removes the mechanical feature of the interface support region **14,** in this case a snap-fit tab **24,** that fastens the ultrasound transducer **40** to the ultrasound coupling adapter **10.** The removal of the mechanical feature of the interface support region **14** dislodges the ultrasound transducer **40** from the coupling device.

The ultrasound coupling adapter **10** cross-sectional shape may be selected from, but not limited to, the group comprising: a circle, square, oval, hexagon, octagon, rectangle, triangle, or any other shape to match or accept an ultrasound transducer **40** of various shapes and sizes. The ultrasound coupling adapter **10** material composition may be comprised of, but not limited to, polyethylene, polypropylene, ABS, polycarbonate, silicone, or santoprene. The ultrasound coupling adapter **10** could be made from a stiff material for treatment in flat areas. The ultrasound coupling adapter **10** may also be made from a flexible or pliable material for conformance to curved or concave areas of treatment. The ultrasound coupling adapter **10** may also be designed with reliefs or unique cross-sections that allow compliance and are independent of material selection.

Figures 2A - 2C show renderings of the embodiment from Figure 1. Figure 2A represents one embodiment of the ultrasound coupling device **50** during storage and/or shipping. The foil seals **11** seal the ultrasound coupling medium **30** in a water-tight chamber. When the therapy is being prepared, the foil seals **11** are removed, exposing the ultrasound coupling medium **30** as in Figure 2B. Figure 2C shows an ultrasound transducer **40** mechanically connected to the ultrasound coupling device **50.** The ultrasound coupling device **50** can then be positioned on a patient's body in a location requiring treatment. Alternatively, the ultrasound coupling device **50** of Figure 2B may be positioned on a patient's body in a location requiring treatment followed by connection of the ultrasound transducer **40** to the ultrasound coupling device **50.**

Figures 3A - 3C show illustrations and drawings depicted various shapes of the ultrasound coupling device **50** adhesive fabric **80** as disclosed in Figure 1. Figure 3A depicts a four-lobed symmetric adhesive fabric **80.** The lobes create a larger surface area for adherence of the ultrasound coupling device **50** to the patient's body. The symmetry of the shape is typically used on flat or larger areas of the body, but may be used everywhere. Figure 3B depicts a "Y" shaped adhesive fabric **80.** This shape is typically used in asymmetric surfaces and on joints. The tail of the "Y" is placed on the articulating surfaces of the joint. Figure 3C depicts a modified "Y" shaped adhesive fabric **80.** The bottom or tail portion of the "Y" is split into two strips and is placed on the articulating surfaces of the joint. The "Y" shaped adhesive fabrics **80** provide relief when moving the joints and prevent pulling and discomfort while treating the joints.

Figures 4A - 4B show renderings of two embodiments of the ultrasound coupling adapter **10** with two methods for mechanically connecting the coupling bandage to an ultrasound transducer **40** by way of the interface support region **14.** Figure 4A depicts a mechanical snap-fit tab **24** connection on the inner portion of the ultrasound coupling adapter **10.** The ultrasound transducer **40** is then connected internally within the inner chamber **12.** Figure 4B depicts a mechanical snap-fit tab **24** connection on the outer portion of the ultrasound coupling adapter **10.** The ultrasound treatment is then connected externally to the inner chamber **12.** Figure 4A and 4B depict a two-way mechanical connection where the ultrasound transducer **40** may be connected and disconnected. However, another embodiment may comprise hooked or angled snap-fit tab **24** projections which serve as a one-way mechanical connection.

Figure 5 is an illustration of the ultrasound coupling device **50** that may be mechanically coupled to an ultrasound transducer **40.** In turn, the ultrasound transducer **40** is mechanically coupled to a wrap **13,** adhesive fabric **80,** brace **15,** or other fixturing device. Alternatively, the ultrasound coupling device **50** may mechanically couple to both the ultrasound transducer **40** and either a wrap **13,** adhesive fabric **80,** brace **15,** or other fixturing device by way of the interface support region **14.** One embodiment of the ultrasound coupling device **50** comprises a top foil seal **11,** ultrasound coupling adapter **10,** ultrasound coupling medium **30,** and bottom foil seal **11,** similar to that disclosed in Figure 1. The ultrasound coupling adapter **10** houses, and provides structure to, the ultrasound coupling medium **30.** The foils seals **11** seal the inner chamber **12** and create a water-tight barrier to the atmosphere during storage and shipping of the ultrasound coupling device **50.**

Another embodiment of the ultrasound coupling device **50** comprises a ultrasound coupling adapter **10** and ultrasound coupling medium **30.** The ultrasound coupling adapter **10** provides mechanical stability and may have mechanisms to prevent repeated use of the ultrasound coupling device **50,** such as pull tab **22** or other mechanism disclosed in this invention. Evaporation of water in different ultrasound coupling media **30** would cause shriveling of the media and prevent further use of the ultrasound coupling device **50** in treatment.

Figures 6A - 6C are renderings of one embodiment of the limited use ultrasound coupling adapter **10** coupled to an ultrasound transducer **40** that uses a mechanical means for rendering the ultrasound coupling system **60** inoperable. Figure 6A depicts an ultrasound transducer **40** mechanically connected to one embodiment of the ultrasound coupling adapter **10.** A perforated of thinned area **23** is shown at 1/3 the height of the ultrasound coupling adapter **10.** The perforated or thinned area **23** may appear anywhere on the height of the ultrasound coupling adapter **10.** Furthermore, the perforated or thinned area **22** may extend completely or partially around the circumference of the ultrasound coupling adapter **10.** In embodiments where the ultrasound coupling adapter **10** is not circular, the perforated or thinned area **22** may extend completely or partially around the perimeter of the ultrasound coupling adapter **10.** In another embodiment, the perforated or thinned area **22** may not be confined to a single plane and may curve upward or downward to complete the destruction of the ultrasound coupling adapter **10.**

Figure 6B is a cross-section of the ultrasound coupling adapter **10** connected to the ultrasound transducer **40.** A one-directional mechanical snap-fit tab **24** is shown as the mechanical method for connecting the ultrasound coupling adapter **10** to the ultrasound transducer **40.** In this case, a method of removing the ultrasound transducer **40** by destroying the ultrasound coupling adapter **10** is required. In another embodiment, the mechanical snap-fit tab **24** may be two-directional, allowing for the ultrasound transducer **40** to be removed.

Figure 6C is a cross-section of the ultrasound coupling adapter **10** parallel to the top surface of the ultrasound coupling adapter **10.** The cross-section is taken at the leverage point for breaking the ultrasound coupling adapter **10.** A slotted portion, otherwise known as a keyseat **27,** on the external surface of the ultrasound coupling adapter **10** fits a boss, otherwise known as a key **26,** from the ultrasound transducer **40.** Once the ultrasound transducer **40,** or ultrasound coupling adapter **10,** is rotated in relation to the other, the key **26** of the ultrasound transducer **40** is pressed against the end of the keyseat **27** in the ultrasound coupling adapter **10,** which creates a shear force in the perforated or thinned area **23.** The shear force allows for the breaking of the ultrasound coupling adapter **10,** rendering the ultrasound coupling adapter **10,** and thus the ultrasound coupling device **50** inoperable. The breaking of the ultrasound coupling adapter **10** further permits removal of the ultrasound transducer **40** for subsequent use with new ultrasound coupling adapters **10.** In another embodiment, the ultrasound transducer **40** may have the keyseat **27** while the ultrasound coupling adapter **10** comprises the key **26** for fitting into the keyseat **27** and creating the leverage point for breaking the ultrasound coupling adapter **10.** Furthermore, the leverage point, i.e. the key **26** and keyseat **27** pair, may be anywhere on the height of the ultrasound coupling adapter **10** or even on the base of the ultrasound coupling adapter **10** (i.e. opposite the patient contacting surface).

Figure 7 is one embodiment of the present invention utilizing a mechanical means to render the ultrasound coupling adapter **10** inoperable. The ultrasound coupling adapter **10** comprises an interface support region **14** that consists of four snap-fit tabs **24.** The snap-fit tab **24** feature allows connection to the ultrasound transducer **40.** These four snap-fit tabs **24** are surrounded by thinned or perforated areas **23.** When the ultrasound transducer **40** is removed, the upward pull of the ultrasound transducer **40** imparts a transverse force onto the snap-fit tabs **24.** The transverse force is large enough to break the thinned or perforated areas **23** surrounding the snap-fit tabs **24.** This breaks the interface support region **14,** and thus the mechanical connection, freeing the ultrasound transducer **40,** and rendering any further uses of the ultrasound coupling adapter **10** futile. The number of snap-fit tabs **24** may increase or decrease in quantity or size depending on the strength of connection required and decoupling force desired.

Figure 8 is one embodiment of the present invention utilizing a perforated or thinned area **23** that is located on the base of the ultrasound coupling adapter **10** (i.e. the portion that contacts the patient's body). The thinned or perforated area **23** may be present at any location on the base and may extend completely or partially around the perimeter of the ultrasound coupling adapter **10.** In one embodiment, bosses on the ultrasound transducer **40** and ultrasound coupling adapter **10** interface support region **14** intertwine with each other, like cogs in a gear, and create a leverage point for breaking the thinned or perforated area **23** when the ultrasound transducer **40** or ultrasound coupling adapter **10** is rotated in relation to the other.

Figure 9 is one embodiment of the present invention that incorporates both mechanical and electrical safety features. The ultrasound coupling adapter **10** consists of a threaded region **17** and vertical flange **16.** An ultrasound transducer **40** is screwed onto the ultrasound coupling adapter **10** to bring the ultrasound transducer **40** in contact with the ultrasound coupling medium **30.** When the ultrasound transducer **40** is completely screwed into place, a boss on the ultrasound transducer **40** dislodges the vertical flange **16** from the ultrasound coupling adapter **10,** which angles the flange **16** and brings it into contact with a tactile switch **76** located within the ultrasound transducer **40.** The depression of the tactile switch **76** either completes or breaks an internal circuit located on a printed circuit board (PCB) **18** within the ultrasound transducer **40** signaling that treatment may be administered safely. When the ultrasound transducer **40** is removed, the flange **16** on the ultrasound coupling adapter **10** is permanently deformed, inhibiting any further use of the ultrasound coupling adapter **10** in subsequent treatments. The activation switch **76** may further be selected from the group of switches comprising slide, toggle, rotary, rocker, knife, pushbutton, membrane, optical, infrared, or the like.

Figure 10 is one embodiment of the ultrasound ultrasound coupling system **60** that includes a switch **76** embedded in the ultrasound transducer **40.** The switch is located above the ultrasound coupling medium **30** of the ultrasound coupling device **50.** When the ultrasound transducer **40** is mechanically connected to the ultrasound coupling adapter **10,** as disclosed in this invention, the switch **76** is depressed by the ultrasound coupling medium **30.** The ultrasound coupling medium **30** is comprised of a hydrogel or other semi-solid or solid material that allows efficient transmission of ultrasound. The stiffness of the ultrasound coupling medium **30** allows for the depression of the switch **76** located in the ultrasound transducer **40.** Once the switch **76** is depressed, a PCB **18** circuit is either connected or broken, allowing for the activation of a treatment. As the ultrasound coupling medium **30** dries and shrinks, the contact to the switch **76** is lost and further treatment cannot be activated.

In another embodiment of the present invention, Figure 11 depicts an electromechanical means **70** to activate an ultrasound transducer **40** using a switch **76** that is embedded in the ultrasound transducer **40** and is situated above the ultrasound coupling adapter **10** sidewall. When the ultrasound transducer **40** is mechanically connected to the ultrasound coupling adapter **10** by way of the interface support region **14,** the ultrasound coupling adapter **10** depresses the switch **76** on the ultrasound transducer **40** allowing for activation of an ultrasound treatment. The switch **76** may be present at any location in the ultrasound transducer **40** that is situated above the perimeter of the ultrasound coupling adapter **10.**

In another embodiment of the present invention, Figure 12 depicts a switch **76** located between the PCB **18** and the ultrasound transducer **40.** The ultrasound transducer **40,** or the lens **33** or enclosure in which the ultrasound transducer **40** is affixed, is allowed to translate more proximal or more distal to the mounted switch **76.** The ultrasound coupling device **50** comprises the ultrasound coupling adapter **10,** for mechanically connecting the ultrasound transducer **40,** and an ultrasound coupling medium **30.** The ultrasound coupling medium **30** may be stiff or slightly compressible, such as with hydrogels, or other solid or semi-solid materials that are transmissive to ultrasound. The stiffness of the ultrasound coupling medium **30** imparts force on the ultrasound transducer **40,** or the lens **33** or enclosure in which the ultrasound transducer **40** is affixed, causing translation toward the mounted switch **76** in the ultrasound transducer **40.** The translation of the ultrasound transducer **40** depresses the switch **76** and allows activation of an ultrasound treatment. Alternatively, the ultrasound coupling adapter **10** of the ultrasound coupling device **50** may have a mechanical boss that imparts a force on the lens **33,** enclosure, or ultrasound transducer **40** of the and similarly cause depression of the switch **76.** With a mechanical boss that translates the lens **33,** enclosure, or ultrasound transducer **40** of the, ultrasound coupling media **30** of any viscosity or stiffness may be used. Furthermore, upon removal of the ultrasound coupling adapter **10** from the ultrasound transducer **40,** the mechanical boss may be permanently deformed, such that reconnection of the ultrasound transducer **40** with the same ultrasound coupling adapter **10** does not activate treatment.

Figure 13 depicts one embodiment of the present invention that includes an ultrasound transducer **40,** ultrasound coupling adapter **10,** and a third connecting component **34.** The ultrasound transducer **40** is mechanically connected to the ultrasound coupling adapter **10** using methods disclosed in the current invention. A third connecting component **34** is inserted into the assembly that further connects the ultrasound transducer **40** and ultrasound coupling adapter **10.** The connectingcomponent **34** also acts as a means to activate the ultrasound treatment. In one embodiment, the connecting component **34** depresses a mechanical switch **76** on the ultrasound transducer **40** once inserted. Depression of the switch **76** either breaks or connects an internal circuit that allows activation of the treatment. In another embodiment, the connecting component **34** may be metallic or electrically conductive to electrically connect two or more contacts **21** located on the ultrasound transducer **40.** In yet another embodiment, the connecting component **34** may be a flange with living hinge that is integral with the ultrasound coupling adapter **10** of the. The ultrasound coupling adapter **10** limits the use of the ultrasound transducer **40** by requiring that the connecting component **34** be removed to begin another treatment. Upon removal, the connecting component **34,** or the ultrasound coupling adapter **10,** is damaged and inhibits further treatment using the same ultrasound coupling adapter **10.**

Figure 14 shows an ultrasound coupling adapter **10** with embedded metallic or electrically conductive ring **29.** The conductive ring **29** may extend completely or partially around the perimeter of the ultrasound coupling adapter **10.** The conductive ring **29** may further be located at any dimension along the height of the ultrasound coupling adapter **10.** When the ultrasound transducer **40** is connected to the ultrasound coupling adapter **10,** electrical means **72** are used to either activate the treatment, in the form of two or more contacts **21** on the ultrasound transducer **40** electrically connected by the conductive ring **29** on the ultrasound coupling adapter **10.** The contacts **21** are electrically connected to a PCB **18** with or without the use of wires **19.** The connection of the two or more contacts **21** on the ultrasound transducer **40** completes a circuit internal to the ultrasound transducer **40** that allows activation of the ultrasound treatment. In another embodiment, the electrically conductive ring **29** may be any shape that completely or partially extends around the perimeter of a ultrasound coupling adapter **10.**

Figure 15 depicts one embodiment of the present invention where a switch **76** is mounted inside the ultrasound transducer **40.** The location of the switch **76** is such that when the ultrasound transducer **40** is mechanically connected to the ultrasound coupling adapter **10,** the switch **76** is depressed by the base of the ultrasound coupling adapter **10** (i.e. by the portion of the ultrasound coupling adapter **10** extending radially out from the chamber sidewall). In the embodiment shown in Figure 15, the switch **76** contacts the surface of the ultrasound coupling adapter **10** base. When the switch **76** is depressed, an internal circuit to the ultrasound transducer **40** is either broken or completed, which allows activation of the ultrasound treatment. In another embodiment, the switch **76** may be depressed by an adhesive fabric **80** or by the patient surface.

Figure 16 depicts the use of wireless methods to communicate, and activate, an ultrasound transducer **40.** One embodiment of the ultrasound coupling adapter **10** comprises an ultrasound coupling adapter **10** with or without foil seals **11,** ultrasound coupling medium **30,** and a adhesive fabric **80.** The adhesive fabric **80** is composed of four layers of material. The first and second layers are a non-woven or similar fabric. The third layer is an adhesive layer that permits attachment to a patient's body. The adhesive may comprise acrylics, silicones, or the like. The fourth layer is a paper liner that prevents premature adhesion to other materials. In between the first and second layer, at least one electrical component is deposited. The electrical component may consist of a Near Field Communication (NFC) tag **31,** Radio Frequency Identification (RFID) tag, Bluetooth module, Bluetooth Low Energy (BLE) module, Wireless Fidelity (Wi-Fi) module, Zigbee module, cellular module, cloud module, or other wireless communication modules, a power source, memory, or the like. The adhesive fabric **80** may have more or less than four layers and the at least one electrical component may be deposited at any location in between any two layers or on top or bottom of the adhesive fabric **80.**

In one embodiment, an NFC tag **31** is used within the adhesive fabric **80** and is positioned in such a way that it is in close proximity to the coupled ultrasound transducer **40.** The NFC tag **31** may be active, requiring a power source, or passive, only requiring the tag itself. The NFC tag **31** may contain a predetermined access code that, once read by the NFC reader **32** technology embedded in the ultrasound transducer **40,** will verify the access code and permit activation of the ultrasound treatment. The access code may be similar across all ultrasound coupling devices **50,** or may have a unique access code for each ultrasound coupling device **50.** With unique access codes, the NFC reader **32** will ensure that no duplicate ultrasound coupling devices **50** may be used past the predetermined life of the ultrasound coupling device **50.** Unique access codes may further be transmitted and checked against a cloud database of codes. With a generalized access code for all ultrasound coupling devices **50,** an NFC write operation from the NFC reader **32** located in the ultrasound transducer **40** can erase the access code in the NFC tag **31** following a successful read of the NFC tag **31** and activation of the ultrasound treatment. With the NFC tag **31** erased, the ultrasound coupling device **50** could not be used in further treatments. The power source used for active tags and devices may consist of lithium polymer, lithium ion, nickel cadmium, or other common battery types. The power source may further consist of devices that harvest electrostatic potentials on a patient's body or devices that use movement or temperature of a patient's body to develop a potential to power electrical components.

In another embodiment, a Bluetooth low energy (BLE) module deposited on the ultrasound coupling adapter **10** may communicate with the ultrasound transducer **40** to activate the ultrasound treatment. Additionally, a main control module of the ultrasound transducer **40** may be used as the activation point for ultrasound treatment.

In another embodiment of the present invention, Figure 17 depicts an NFC tag **31,** RFID tag, or other wireless communication modules embedded within the inner chamber **12** or within the ultrasound coupling adapter **10.** An NFC tag **31** may be overmolded with a plastic such that the ultrasound coupling adapter **10** is formed through injection molding with an embedded tag. Besides the location and manufacturing method for positioning a tag within the inner chamber **12** or ultrasound coupling adapter **10,** the basic mechanism of use is similar to Figure 16.

Figure 18 depicts an electrical means **72** of analyzing the ultrasound coupling medium **30** of the ultrasound coupling device **50** to determine if further treatment is safe. The ultrasound transducer **40** consists of two electrical contacts **21** that interact with the ultrasound coupling medium **30.** A series of low current sinusoidal waves are swept through a number of frequencies related to the ultrasound frequency delivered. By sweeping the frequencies and analyzing the voltage and/or current differences, the electrical impedance of the ultrasound coupling medium **30** can be discerned. Based on preset values, the ultrasound transducer **40** would prevent activation of a treatment if the electrical impedance is below the preset value. Alternatively, the swept frequencies may be unrelated to the frequency of ultrasound used in the treatment.

In another embodiment, the sinusoidal wave used to drive the ultrasound transducer **40** is swept through different frequencies. Depending on the voltage and current values measured during the different frequencies, the electrical impedance of the ultrasound transducer **40,** and thus the acoustic impedance of the system, can be determined and compared against preset values. If the electrical/acoustic impedance is determined to be lower than the preset value, further treatments would be prevented using the current ultrasound coupling device **50.**

One embodiment of the present invention comprises a ultrasound coupling adapter **10** with at least one embedded electrical component. The at least one electrical component consists of other safety and functionality mechanisms that would limit the furtherance of treatment using the current ultrasound coupling adapter **10.** In one embodiment, a thermocouple or temperature sensor is embedded into the ultrasound coupling adapter **10.** The rate of heating, maximum temperature, and time above a predetermined temperature may be tracked within the ultrasound coupling adapter **10** or the data transmitted to the ultrasound transducer **40** or ultrasound control module. In ultrasound coupling devices **50** with less effective transmission of ultrasound energy, more heat would be dissipated and would be measured by the temperature sensor embedded within the ultrasound coupling adapter **10.** The ultrasound transducer **40** would compare the temperature measurements to preset values and would limit further treatment using the current ultrasound coupling adapter **10.**

One embodiment of the present invention involves a chemical means to limit the use of the ultrasound coupling device **50.** The ultrasound coupling device **50** comprises an ultrasound coupling adapter **10,** ultrasound coupling medium **30,** and the option of foil seals **11.** The ultrasound coupling medium **30** chemical composition is such that degradation of the ultrasound coupling medium **30** occurs after a prescribed time of use. The degradation may be activated or assisted through increased heat, ultrasound energy, a chemical compound, or a combination of the aforementioned. In one embodiment, the ultrasound coupling medium **30** is composed of two or more layers that are individually transmissive to acoustic energy. Upon activating the ultrasound treatment, mixing of the chemicals occurs, through ultrasound-assisted acoustic streaming, causing the ultrasound coupling medium **30** to shrink in volume and the ultrasound coupling device **50** to become inoperable. In another embodiment, the ultrasound coupling medium **30** volume remains the same, but the molecular structure changes causing inefficient transmission of acoustic energy, which warrants changing the ultrasound coupling device **50.** The change in molecular structure may be signaled by a color or opacity change that signals the user to change the ultrasound coupling device **50.**

In yet another embodiment, the ultrasound coupling adapter **10** comprises a material, such as PLA, PLGA, or the like, that degrades through chemical, thermal, or ultrasound-assisted means. The design and material composition of the ultrasound coupling adapter **10** may be designed such that the degradation time is predetermined. When the ultrasound coupling adapter **10** degrades, the integrity of the interface support region **14** is compromised and the mechanical connection to an ultrasound transducer **40** is prevented.

Another embodiment of the present invention is an ultrasound coupling adapter **10** with a magnetic source embedded within the adhesive fabric **80.** When the ultrasound transducer **40,** or control module, is connected to, or brought in close proximity to, the ultrasound coupling adapter **10,** respectively, a circuit senses the magnetic energy and allows activation of the ultrasound treatment. In another embodiment, the magnetic source may be placed within the inner chamber **12** or embedded within the ultrasound coupling adapter **10.**

In another embodiment, an adhesive fabric **80** may be used to couple the ultrasound coupling adapter **10** to a location on the patient's body. After one or a predefined number of treatments, the adhesive properties of the adhesive fabric **80** substantially diminish to prevent further coupling of the ultrasound coupling adapter **10** to the patient. In one embodiment, the adhesive properties of the adhesive fabric **80** are diminished by 40% or greater after one use according to the ASTM D903 standard.

One embodiment of the present invention is a method of using an optical means of limiting the use of the ultrasound coupling device **50.** The ultrasound coupling device **50** consists of an ultrasound coupling adapter **10,** ultrasound coupling medium **30,** and an adhesivefabric **80.** A barcode or Quick Response (QR) code may be embedded on the adhesive fabric **80** or ultrasound coupling adapter **10.** When the ultrasound transducer **40** is connected to the ultrasound coupling adapter **10,** a barcode of QR code reader visually identifies the code of the ultrasound coupling device **50** and permits activation of the ultrasound treatment if the code is found in a database or is not identical to a previously scanned code. The scanning of the code may also be completed before mechanical connection of the ultrasound transducer **40** to the ultrasound coupling adapter **10.** Alternatively, the ultrasound control module may read the code and subsequently permit activation of the ultrasound transducer **40.** Additionally, the ultrasound coupling adapter **10** may comprise foilseals **11** that include a barcode or QR code for activation of the ultrasound coupling device **50.** After scanning of the code and activation of the treatment, an internal microchip of the ultrasound transducer **40** may limit the use of the ultrasound coupling device **50** to a predetermined period time and prevent further activation of the treatment with the same code.

In another embodiment, an optical switch is located in the ultrasound transducer **40.** When the ultrasound transducer **40** is mechanically connected to the ultrasound coupling adapter **10,** the optical switch is interrupted. This interruption signals to the ultrasound transducer **40** that treatment activation is safe. Once removed, the ultrasound coupling adapter **10** may permanently deform, which prevents interruption of the optical switch in subsequent attempts to reuse the ultrasound coupling adapter **10.**

In another embodiment, an optical switch is located in the ultrasound transducer **40.** When the ultrasound transducer **40** is mechanically connected to the ultrasound coupling adapter **10,** the optical switch is completed. This may be accomplished by a spring or flange that normally interrupts the optical switch, but is altered upon connection to the ultrasound coupling adapter **10,** to allow completion of the optical switch. In another embodiment, the ultrasound coupling adapter **10** may comprise a reflective or angled surface that bends or reflects the light and completes the optical switch. When the ultrasound coupling adapter **10** is removed from the ultrasound transducer **40,** the ultrasound coupling adapter **10** is permanently deformed, which prevents the completion of the optical switch in subsequent attempts to reuse the ultrasound coupling adapter **10.**

In another embodiment, an optical sensor may be located within the ultrasound transducer **40.** The optical sensor may emit light and record the index of refraction of the ultrasound coupling medium **30.** Once the ultrasound coupling adapter **10** is connected to the ultrasound transducer **40,** the optical sensor is able to measure the index of refraction and compare it to preconfigured limits. As the ultrasound coupling device **50** is used, the moisture content of the ultrasound coupling medium **30** will decrease and the index of refraction will change. When the limit is reached, the ultrasound transducer **40** will not activate until a new ultrasound coupling device **50** is used. In a similar embodiment, the reflection, or backscattering, of light is measured by the optical sensor. As the ultrasound coupling device **50** is used and the moisture content of the ultrasound coupling medium **30** decreases, the light reflection and/or back-scatter will increase. The measured values are checked against predetermined values and the activation of treatment is prevented when the limit is reached.

In another embodiment, an infrared sensor located in the ultrasound transducer **40** measures the temperature profile of the ultrasound coupling medium **30.** The sensor records the maximum temperature, rate of heating, and time spent above a certain temperature to determine the safety and effectiveness of the ultrasound coupling medium **30.** Based on predetermined values, the activation of ultrasound is prevented when the limit is reached.

In another embodiment, an optical sensor located in the ultrasound transducer **40** measures the color or opacity of the ultrasound coupling medium **30.** As the ultrasound coupling device **50** is used, the moisture content of the ultrasound coupling medium **30** is reduced and the color and/or opacity of the ultrasound coupling medium **30** is changed. This change is measured by the optical sensor and limits the activation of treatment. The color/opacity limit may be a predetermined absolute value or a ratio or percentage of the initial value compared to the final value.

In another embodiment, an oxygen or ultrasound-sensitive chemical or molecule is added to the ultrasound coupling medium **30.** The molecule does not interrupt the transmission of ultrasound, but changes the color or opacity of the ultrasound coupling medium **30** after a predetermined exposure to ultrasound and/or oxygen. The change in color or opacity is measured by an optical sensor and the value is compared to a database. When a limit is reached, the ultrasound treatment is prevented until a new ultrasound coupling device **50** is used.

While several aspects of the present invention have been described and depicted herein, alternative aspects may be effected by those skilled in the art to accomplish the same objectives. Accordingly, it is intended by the appended claims to cover all such alternative aspects as fall within the scope of the invention.

## Claims

1. An ultrasound coupling device (50) comprising:
an ultrasound coupling adapter (10) for coupling an ultrasound transducer (40) to an ultrasound coupling medium (30) housed in the ultrasound coupling adapter (10), said ultrasound coupling adapter (10) comprising:
an interface support region (14) for operably interfacing the ultrasound transducer (40) to the ultrasound coupling medium (30); and
an integrated means (22, 23, 24, 26, 27) for rendering the ultrasound coupling adapter (10) inoperable, thereby rendering the ultrasound coupling device inoperable,
wherein the integrated means (22, 23, 24, 26, 27) functions to permanently alter the interface support region (14),
wherein the integrated means (22, 23, 24, 26, 27) consists of a perforated or thinned area (23) and a means selected from the group consisting of a pull tab (22), a snap-fit tab (24), and a stress concentrator (26, 27), said integrated means (22, 23, 24, 26, 27) being configured to facilitate permanently altering a portion of the interface support region (14),
wherein the perforated or thinned area (23) completely or partially extends around the perimeter of the interface support region (14) of the ultrasound coupling adapter (10).

2. The ultrasound coupling device (50) according to claim 1, wherein the pull tab (22) serves as a lever capable of rendering the ultrasound coupling device (50) inoperable.

3. The ultrasound coupling device (50) according claim 1 or 2, wherein the interface support region (14) comprises a key (26) and keyseat (27) pair configuration with an ultrasound transducer (40) that interface together to facilitate the permanent altering of the configuration, or
wherein the interface support region (14) comprises a threaded portion (17) that couples with an ultrasound transducer (40), and wherein the threaded portion (17) prevents decoupling with the ultrasound transducer (40) unless at least a portion of said interface support region (14) is permanently altered.

4. The ultrasound coupling device (50) according to claim 1 further comprising an adhesive fabric (80) for interfacing the ultrasound coupling device (50) with a subject,
wherein the fabric (80) has adhesive properties that substantially diminish after first use by the subject or
wherein the fabric (80) has adhesive properties that diminish by 50% or greater using the ASTM D903 standard.

5. An ultrasound coupling system (60) comprising:
- an ultrasound coupling device (50) according to any one of claims 1-4; and
- an ultrasound transducer (40) configured for operable attachment to the ultrasound coupling device (50).

6. A method of regulating application of ultrasound energy to a subject, said method comprising the steps of:
- applying ultrasound energy to a subject using an ultrasound coupling system (60) according to claim 5; and
- manipulating the integrated means (22, 23, 24, 26, 27) of the ultrasound coupling adapter (10) so as to render the ultrasound coupling device (50) inoperable, thereby causing the ultrasound energy to cease being applied to the subject, wherein the manipulating step is performed by the subject and comprises the permanent altering of the interface support region (14).

## Patentansprüche

1. Ultraschallkopplungsvorrichtung (50), Folgendes umfassend:
einen Ultraschallkopplungsadapter (10) zum Koppeln eines Ultraschallwandlers (40) mit einem Ultraschallkopplungsmedium (30), das in dem Ultraschallkopplungsadapter (10) untergebracht ist, wobei der Ultraschallkopplungsadapter (10) Folgendes umfasst:
eine Schnittstellenstützregion (14) zum betriebsfähigen Verbinden des Ultraschallwandlers (40) mit dem Ultraschallkopplungsmedium (30); und
ein integriertes Mittel (22,23, 24, 26, 27) zum Außerbetriebsetzen des Ultraschallkopplungsadapters (10), wobei dadurch die Ultraschallkopplungsvorrichtung außer Betrieb gesetzt wird,
wobei das integrierte Mittel (22, 23, 24, 26, 27) arbeitet, um die Schnittstellenstützregion (14) dauerhaft zu ändern,
wobei das integrierte Mittel (22, 23, 24, 26, 27) aus einem perforierten oder einem ausgedünnten Bereich (23) und einem Mittel besteht, das aus der Gruppe ausgewählt ist, die aus einer Zuglasche (22), einer Schnapplasche (24) und einem Spannungskonzentrator (26, 27) besteht, wobei das integrierte Mittel (22, 23, 24, 26, 27) konfiguriert ist, um das dauerhafte Ändern eines Abschnitts der Schnittstellenstützregion (14) zu ermöglichen,
wobei sich der perforierte oder derausgedünnte Bereich (23) vollständig oder teilweise um den Umfang der Schnittstellenstützregion (14) des Ultraschallkopplungsadapters (10) erstreckt.

2. Ultraschallkopplungsvorrichtung (50) nach Anspruch 1, wobei die Zuglasche (22) als ein Hebel dient, der in der Lage ist, die Ultraschallkopplungsvorrichtung (50) außer Betrieb zu setzen.

3. Ultraschallkopplungsvorrichtung (50) nach Anspruch 1 oder 2, wobei die Schnittstellenstützregion (14) eine Keil- (26) und Keilnut-(27)Paarkonfiguration mit einem Ultraschallwandler (40) umfasst, die miteinander verbunden sind, um das dauerhafte Ändern der Konfiguration zu ermöglichen, oder
wobei die Schnittstellenstützregion (14) einen Gewindeabschnitt (17) umfasst, der mit einem Ultraschallwandler (40) koppelt, und wobei der Gewindeabschnitt (17) ein Entkoppeln mit dem Ultraschallwandler (40) verhindert, sofern nicht wenigstens ein Abschnitt der Schnittstellenstützregion (14) dauerhaft geändertwird.

4. Ultraschallkopplungsvorrichtung (50) nach Anspruch 1, ferner umfassend ein Klebstoffgewebe (80) zum Verbinden der Ultraschallkopplungsvorrichtung (50) mit einem Subjekt,
wobei das Gewebe (80) Klebstoffeigenschaften aufweist, die nach einer ersten Verwendung durch das Subjekt im Wesentlichen abnehmen, oder
wobei das Gewebe (80) Klebstoffeigenschaften aufweist, die unter Verwendung der ASTM D903-Norm um 50 % oder mehr abnehmen.

5. Ultraschallkopplungssystem (60), Folgendes umfassend:
- eine Ultraschallkopplungsvorrichtung (50) nach einem der Ansprüche 1-4; und
- einen Ultraschallwandler (40), der für eine betriebsfähige Befestigung an der Ultraschallkopplungsvorrichtung (50) konfiguriert ist.

6. Verfahren zum Regulieren einer Anwendung von Ultraschallenergie auf ein Subjekt, wobei das Verfahren die folgenden Schritte umfasst:
- Aufbringen von Ultraschallenergie auf ein Subjekt unter Verwendung eines Ultraschallkopplungssystems (60) nach Anspruch 5; und
- Manipulieren des integrierten Mittels (22, 23, 24, 26, 27) des Ultraschallkopplungsadapters (10), um die Ultraschallkopplungsvorrichtung (50) außer Betrieb zu setzen, wobei dadurch bewirkt wird, dass die Ultraschallenergie nicht mehr auf das Subjekt aufgebracht wird, wobei der Manipulationsschritt durch das Subjekt durchgeführt wird und das dauerhafte Ändern der Schnittstellenstützregion (14) umfasst.

## Revendications

1. Dispositif de couplage à ultrasons (50) comprenant :
un adaptateur de couplage à ultrasons (10) pour couplerun transducteur à ultrasons (40) à un milieu de couplage à ultrasons (30) logé dans l'adaptateur de couplage à ultrasons (10), ledit adaptateur de couplage à ultrasons (10) comprenant :
une région de support d'interface (14) pour interfacer de manière fonctionnelle le transducteur à ultrasons (40) au milieu de couplage à ultrasons (30) ; et
un moyen intégré (22, 23, 24, 26, 27) pour rendre inopérant l'adaptateur de couplage à ultrasons (10), rendant ainsi le dispositif de couplage à ultrasons inopérant, dans lequel le moyen intégré (22, 23, 24, 26, 27) fonctionne pour modifier de manière permanente la région de support d'interface (14),
dans lequel le moyen intégré (22, 23, 24, 26, 27) est constitué d'une zone perforée ou amincie (23) et un moyen choisi dans le groupe constitué d'une languette (22), d'une languette à encliquetage (24) et d'un concentrateur de contraintes (26, 27), ledit moyen intégré (22, 23, 24, 26, 27) étant conçu pour faciliter la modification permanente d'une partie de la région de support d'interface (14),
dans lequel la zone perforée ou amincie (23) s'étend complètement ou partiellement autour du périmètre de la région de support d'interface (14) de l'adaptateur de couplage à ultrasons (10).

2. Dispositif de couplage à ultrasons (50) selon la revendication 1, dans lequel la languette (22) sert de levier capable de rendre le dispositif de couplage à ultrasons (50) inopérant.

3. Dispositif de couplage à ultrasons (50) selon la revendication 1 ou 2, dans lequel la région de support d'interface (14) comprend une configuration de paire de clavette (26) et de rainure de clavette (27) avec un transducteur à ultrasons (40) qui s'interfacent ensemble pour faciliter la modification permanente de la configuration, ou
dans lequel la région de support d'interface (14) comprend une partie filetée (17) qui se couple avec un transducteur à ultrasons (40), et dans lequel la partie filetée (17) empêche le découplage avec le transducteur à ultrasons (40) à moins qu'au moins une partie de ladite région de support d'interface (14) soit modifiée en permanence.

4. Dispositif de couplage à ultrasons (50) selon la revendication 1, comprenant en outre un tissu adhésif (80) pour interfacer le dispositif de couplage à ultrasons (50) avec un sujet, dans lequel le tissu (80) a des propriétés adhésives qui diminuent sensiblement après la première utilisation par le sujet ou
dans lequel le tissu (80) a des propriétés adhésives qui diminuent de 50 % ou plus à l'aide de la norme ASTM D903.

5. Système de couplage à ultrasons (60) comprenant :
- un dispositif de couplage à ultrasons (50) selon l'une quelconque des revendications 1 à 4 ; et
- un transducteur à ultrasons (40) conçu pour une fixation fonctionnelle au dispositif de couplage à ultrasons (50).

6. Procédé de régulation de l'application d'énergie à ultrasons à un sujet, ledit procédé comprenant les étapes consistant à :
- appliquer une énergie à ultrasons à un sujet à l'aide d'un système de couplage à ultrasons (60) selon la revendication 5 ; et
- manipuler le moyen intégré (22, 23, 24, 26, 27) de l'adaptateur de couplage à ultrasons (10) de manière à rendre le dispositif de couplage à ultrasons (50) inopérant, provoquant ainsi l'arrêt de l'application de l'énergie à ultrasons au sujet, dans lequel l'étape de manipulation est effectuée par le sujet et comprend la modification permanente de la région de support d'interface (14).
